# EUROPEAN PATENT SPECIFICATION

(11) **EP 1 899 015 B1**
(45) Date of publication and mention of the grant of the patent: **21.03.2012**
(21) Application number: 06754585.5
(22) Date of filing: 27.06.2006
(51) Int. Cl.: A61K 8/89, A61Q 17/04, A61K 8/11, A61K 8/35, A61K 8/37

(54) **COMPOSITION WITH TIGHT CAPSULES CONTAINING A SUNSCREEN AGENT**
ZUSAMMENSETZUNG MIT DICHTEN KAPSELN MIT EINEM SONNENSCHUTZMITTEL
COMPOSITION DOTÉE DE CAPSULES ÉTANCHES CONTENANT UN AGENT ÉCRAN SOLAIRE

(30) Priority: 29.06.2005 EP 05014095
(43) Date of publication of application: 19.03.2008
(73) Proprietor: DSM IP Assets B.V., 6411 TE Heerlen (NL)
(72) Inventor: POSCHALKO, Alexander, CH-4127 Birsfelden (CH); BERG-SCHULTZ, Katja, CH-4132 Muttenz (CH); WESTENFELDER, Horst, 67435 Neustadt a. d. W. (DE); MENDROK-EDINGER, Christine, 79618 Rheinfelden-Minseln (DE); ZIMMERMAN, Brett Lee, Frankenmuth, Michigan 48734 (US); SCHIROSI, Henri, B-5070 Vitrival (BE); LABROSSE, Arnaud, B-7170 Manage (BE); MARTEAUX, Léon, B-1160 Bruxelles (BE)
(74) Representative: Best, Michael
(86) International application number: PCT/EP2006/006194
(87) International publication number: WO 2007/000316

(56) References cited:
- EP-A- 1 145 708
- WO-A-00/09652
- WO-A-00/71084
- WO-A-01/24762
- WO-A-01/80823
- WO-A-03/011239
- WO-A-03/041666
- WO-A-2005/009604
- WO-A-2005/053631
- WO-A-2006/061124
- PATENT ABSTRACTS OF JAPAN vol. 1997, no. 09, 30 September 1997 (1997-09-30) & JP 09 118726 A (SOKEN CHEM & ENG CO LTD), 6 May 1997 (1997-05-06)

## Description

The present invention relates to compositions, preferably topical compositions, comprising two different sunscreen agents, one of which is encapsulated in a microcapsule. It was found that a minimum size of the microcapsules is necessary to avoid leakage of the encapsulated sunscreen agent out of the microcapsule. On the other hand, if the microcapsules are too big, they have technological disadvantages, their sunscreen activity is reduced and it is difficult to distribute the microcapsules in a composition uniformly. The present invention thus provides microcapsules containing a certain sunscreen agent and having an average particle size measured as D(v, 0.5) of 3 µm to 5 µm and compositions containing at least two sunscreen agents, at least one of which is encapsulated in microcapsules having an average particle size measured as D(v, 05) of 3 to 8 µm.

There is a constantly increasing need for sunscreen protection agents in a population that is exposed to an increasing amount of damaging sunlight. Repetitive sun exposure can result in skin changes known as photoaged skin. The clinical changes that are seen in photoaged skin differ from those of normally aged skin in sunlight protected sites of the body. Among the damaging results of intensive sun exposure of the skin there is increasing wrinkling, elastosis, pigmentary changes, precancerous and cancerous skin lesions.

Many sunscreen agents have been developed in the past protecting against harmful effects of UV-A (320 to 400 nm) and/or UV-B (290 to 320 nm) wavelength and even shorter wavelength (UV-C). Also broad-spectrum sunscreen agents are known. Sunscreen agents are usually incorporated either alone or in combination with each other into topical cosmetic or pharmaceutical preparations which are widely known and used.

Most sunscreen agents used in such topical compositions are monomeric compounds, and thus, there is the inherent risk that such compounds can penetrate the skin barrier, which is highly undesirable. Sunscreen agents on the basis of polysiloxanes, which may be either linear or cyclic, are described e.g. in WO 93/04665, WO 94/06404, EP-A 0 709 080, WO 92/20690, EP-A 392 883, WO 03/035022, WO 2004/007592, WO 03/086340 and EP-A 358 584. With these polysiloxanes the risk of skin penetration is lower, but it is sometimes difficult to incorporate the polysiloxanes in sunscreen compositions due to compatibility problems which may occur depending on the UV-active chromophores which are covalently bonded to the polysiloxanes.

A significant problem with many of the presently known sunscreen agents is that they tend to interact, which in some cases in which a sunscreen composition contains more than one type of sunscreen agent leads to a situation where the UV filter activity of the sunscreen agents is reduced during storage or after being applied to the skin. Attempts have been made to solve this problem by encapsulating one sunscreen agent or two or more sunscreen agents which are present in sunscreen compositions in order to minimize the contact of the sunscreen agents during storage and when applied to the skin. Microcapsules are disclosed which release the UV filters over time, others are designed to permanently encapsulate the UV filter. Encapsulation technologies are described e.g. in FR 2 642 329, DE-A 195 37 415, EP-A 509 904, FR 2 726 760 and FR 2 687 914 as well as in WO 00/71084, US-A 6,303,149, WO 98/31333, US-A 5,876,699 and WO 00/72806.

US-A 6,303,149, in particular, discloses a process for producing microcapsules with the sunscreen agent having a particle size in the range of 0.01 to 100 µm, preferably of 0.1 to 10 µm. In the examples encapsulated sunscreen agents in the form of microcapsules are produced which have a size of 0.5 to 3.5 µm (example 1), 0.5 to 3 µm (example 3) or 1 to 5 µm (example 4). The particle sizes disclosed in the examples are not mean particle sizes and the D(v, 0.5) values of these exemplified microcapsules are lower than 3 µm.

However, even in those microcapsules which are designed to permanently encapsulate the sunscreen agents, it has been found that there is still a significant amount of leakage of the sunscreen agents through the microcapsules when the microcapsules are incorporated into usual sunscreen compositions. Those microcapsules might be tight, if they are tested in a diffusion test set-up as e.g. described in WO 2005/009604 or in an aqueous environment, however, usual topical sunscreen compositions usually are in the form of an emulsion and contain both hydrophilic and lipophilic compounds (an oil and a water phase) as well as emulsifiers. The inventors of the present invention found that the known microcapsules which were tested to be tight in an aqueous composition or specially designed diffusion test set-ups nevertheless show significant leakage in topical sunscreen compositions which are in the form of emulsions and contain not only aqueous components but also oil components, i.e. hydrophilic and lipophilic compounds and emulsifiers.

Thus, for those cases where a topical composition contains two different sunscreen agents which are not compatible with each other and where the encapsulation of at least one of the sunscreen agents is carried out in order to permanently separate those two incompatible sunscreen agents, there still does not exist a satisfactory encapsulation which prevents leakage of the sunscreen agent through the microcapsule.

These problems of leakage are particularly severe, if the sunscreen agent which is encapsulated is a cinnamic ester derivative such as 2-ethylhexyl methoxycinnamate which is also known as PARSOL^{®} MCX or as EHMC. Cinnamate derivatives such as ethylhexyl methoxycinnamate are known to be useful as sunscreen agents particularly for protecting human skin e.g. in cosmetic compositions. However, those compounds can cause unwanted effects, e.g. allergic reactions, and they have also a significant cross-reactivity with other sunscreen agents, in particular with dibenzoylmethane derivatives such as butyl methoxydibenzoylmethane (also known as 4-tert.-butyl-4'-methoxydibenzoyl-methane e.g. available as PARSOL^{®} 1789 at DSM Nutritional products).

It has been proposed to encapsulate the cinnamate derivatives in various coating matrices which may e.g. be silica or organically-modified silica, and it can be referred e.g. to WO 00/09652, WO 00/71084 and WO 00/72806. Since the cinnamic ester derivatives also undergo photodecomposition during irradiation, which can result in a significant loss of absorbance and, thus, photoprotection. WO 2004/069216 suggests to enhance the photostability of encapsulated cinnamates in topical sunscreen compositions by combining these compounds with at least one additional non-encapsulated sunscreen agent, preferably a UV-B or broad-spectrum sunscreen agent or a combination of a UV-B and/or a UV-A and/or a broad-spectrum sunscreen agent. However, the cinnamate esters are incompatible with many other sunscreen agents, in particular with butyl methoxydibenzoylmethane, which is one of the preferred additional sunscreen agents of WO 2004/069216, and in case of leakage from the microcapsules, both the cinnamate ester and the other sunscreen such as the butyl methoxydibenzoylmethane can be inactivated. This problem may also occur with other usual sunscreen agents. WO 2005/053631 provides microcapsules in which the sunscreen agent is covalently bound to the matrix material.

There is a need in the prior art to provide sunscreen agents in a form which does not show the above problems and in particular to provide compositions, in particular topical compositions, in particular topical pharmaceutical and cosmetic compositions such as sunscreen compositions, which contain two different sunscreen agents which are usually not compatible and which nevertheless retain their activity during storage and after application to the skin to a high degree.

Unexpectedly, it was found that the leakage of sunscreen agents out of microcapsules is dependent on the size of the microcapsules, and if the microcapsules have a certain minimum size, in particular if the microcapsules have a size of at least 3 µm measured as D(v, 0.5), such a leakage does not occur. On the other hand, the microcapsules should not be too big, so that they can easily and effectively be incorporated into compositions, in particular topical cosmetic and pharmaceutical compositions and provide a good sunprotecting activity. The microcapsules disclosed in the prior art usually can have an arbitrary size, and e.g. the microcapsules disclosed in WO 2004/069216 can have a mean diameter of about 0.01 µm to about 100 µm, preferably of 0.1 to 10 µm. However, the microcapsules employed in practice usually are rather small and are in the range of the commercial Eusolex^{®} UV-pearls™ OMC which have a particle size D(v, 0.5) of about 1,34 µm.

The present invention thus in a first aspect provides compositions, preferably topical compositions, comprising a first sunscreen agent which is encapsulated and a second sunscreen agent which is different from the first sunscreen agent, characterized in that the first sunscreen agent is encapsulated in microcapsules having an average particle size D(v, 0.5) in the range from 3 µm to 8 µm wherein the microcapsules are obtainable by an emulsion polymerization process or by a sol-gel process and wherein the encapsulating agent is either in the form of a discrete liquid or a discrete solid. The average particle size D(v, 0.5) of the microcapsules is preferably in the range from 3 to 6 µm, more preferably in the range from 3 to 5 µm and most preferably in the range from 3 to 4 µm.

The compositions of the present invention are preferably topical pharmaceutical or cosmetic compositions and can, of course, contain further sunscreen agents in addition to the two different sunscreen agents of which at least one is encapsulated.

Preferably, at least one of the two different sunscreen agents which are present in the compositions of the present invention is a cinnamic ester derivative of the general formula I 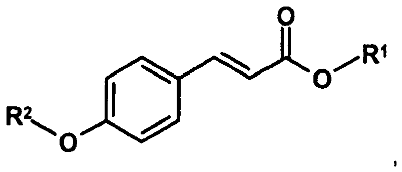 wherein R¹ and R² are independently of each other hydrogen or saturated straight- or branched-chain alkyl containing 1 to 21, preferably 1 to 8 carbon atoms, such as methyl, ethyl, propyl, isopropyl, butyl, sec-butyl, isobutyl, pentyl, neopentyl, hexyl and 2-ethylhexyl. Cinnamate derivatives of formula I which are particularly preferred are 2-ethylhexyl methoxycinnamate (PARSOL^{®} MCX or EHMC), ethoxyethyl methoxycinnamate and isoamyl methoxycinnamate. Most preferred is 2-ethylhexyl methoxycinnamate. While the cinnamic acid derivative can be either the first or the second sunscreen agent of the compositions, it is preferred that the cinnamic ester derivative is the first sunscreen agent of the compositions of the invention, i.e. the sunscreen agent which is encapsulated in the microcapsules.

If the cinnamic ester derivative is the first sunscreen agent, the second sunscreen agent which is present in the topical compositions of the present invention is a sunscreen agent which is not particularly restricted. Preferably, it is a sunscreen agent which is not compatible with the first sunscreen agent. If the first sunscreen agent is a cinnamic acid derivative as defined above, the second sunscreen agent is e.g. selected from PABA, camphor benzalkonium methosulfate, homosalate, benzophenone-3, phenylbenzimidazole sulfonic acid (e.g. PARSOL^{®} HS), terephthalidene dicamphor sulfonic acid (e.g Mexoryl SX), butyl methoxydibenzoylmethane (e.g. PARSOL^{®} 1789), benzylidene camphor sulfonic acid (e.g. Mexoryl SL), octocrylene (e.g. PARSOL^{®} 340), polyacrylamidomethyl benzylidene camphor, PEG-25 PABA, isoamyl p-methoxycinnamate, ethylhexyl triazone (e.g. UVINUL^{®} T150), drometrizole trisiloxane (Mexoryl XL), diethylhexyl butamido triazine (e.g. Uvasorb^{®} HEB), 4-methylbenzylidene camphor (e.g. PARSOL^{®} 5000), 3-benzylidene camphor, ethylhexyl salicylate, ethylhexyl dimethyl PABA, benzophenone-4, methylene-bis-benzotriazolyl tetramethylbutylphenol (e.g. Tinosorb M), disodium phenyl dibenzimidazol tetrasulfonate (Neo Heliopan^{®} AP), bis-ethylhexyloxyphenol methoxyphenyl triazine (Tinosorb^{®} S), polysilicone-15 (PARSOL^{®} SLX), diethylamino hydroxybenzoyl hexyl benzoate (UVINUL^{®} A plus), microfine titanium dioxide (e.g. PARSOL^{®} TX) and microfine zinc oxide. Preferably, the second sunscreen is selected from phenylbenzimidazole sulfonic acid (e.g. PARSOL^{®} HS), butyl methoxydibenzoylmethane (e.g. PARSOL^{®} 1789), octocrylene (e.g. PARSOL^{®} 340), ethylhexyl triazone (e.g. UVINUL^{®} T150), diethylhexyl butamido triazine (e.g. Uvasorb^{®} HEB), methylene-bis-benzotriazolyl tetramethylbutylphenol (e.g. Tinosorb M), disodium phenyl dibenzimidazol tetrasulfonate (Neo Heliopan^{®} AP), bis-ethylhexyloxyphenol methoxyphenyl triazine (Tinosorb^{®} S), polysilicone-15 (PARSOL^{®} SLX), diethylamino hydroxybenzoyl hexyl benzoate (UVINUL^{®} A plus) or microfine titanium dioxide. Most preferably, the second sunscreen agent is butyl methoxydibenzoylmethane, ethylhexyl triazone (e.g. UVINUL^{®} T150), methylene-bis-benzotriazolyl tetramethylbutylphenol (e.g. Tinosorb M), bis-ethylhexyloxyphenol methoxyphenyl triazine (Tinosorb^{®} S) or polysilicone-15 (PARSOL^{®} SLX).

If the cinnamic ester derivative is the second sunscreen agent (i.e. the sunscreen agent which is not necessarily encaosulated in the microcapsules) the above sunscreen agents constitute the first sunscreen agent of the compositions according to the invention (i.e. the sunscreen agent which is encapsulated in the microcapsules).

It is preferred according to the present invention that the first sunscreen agent which is encapsulated is the cinnamic acid derivative as defined above and the second sunscreen agent is as defined above such as butyl methoxydibenzoylmethane.

The second sunscreen agent is preferably present in the compositions according to the present invention in a non-encapsulated form, but it is, of course, also possible to encapsulate both the first and the second sunscreen agent present in the composition of the present invention. If the second sunscreen agent which is present in the composition of the present invention is also encapsulated, the encapsulation of the second sunscreen agent is not particularly restricted, and any known encapsulation can be used.

The compositions comprising the first and second sunscreen agents are preferably topical pharmaceutical or cosmetic compositions, more preferably topical cosmetic compositions. The term topical compositions encompasses compositions such a skin care preparations e.g. body oils, body lotions, body gels, treatment creams, skin protection ointments, shaving preparations, such as shaving foams or gels, skin powders such as baby powder, moisturizing gels, moisturizing sprays, revitalizing body sprays, sunscreen preparations, skin wash preparations such as body wash preparations and hair care preparations such as for example, shampoo, hair conditioners, products for styling and treating hair, perming agents, hair sprays and lacquers, hair gels, hair fixatives and hair dying or bleaching agents.

These topical compositions, in particular the cosmetic compositions, are generally in the form of emulsions or microemulsions, which means that they comprise lipophilic and hydrophilic compounds. These topical compositions contain an oil and a water phase, and according to the present invention such topical compositions in the form of an emulsion or microemulsion containing an oil and a water phase are most preferred. The compositions are particularly suitable for topical applications onto human skin and/or hair.

Thus, the invention also relates to topical pharmaceutical or cosmetic compositions in the form of an emulsion or microemulsion, in particular of an O/W type (oil in water), W/O type (water in oil), O/W/O type (oil-in-water-in-oil) and W/O/W type (water in oil in water). Preferably, the topical pharmaceutical or cosmetic compositions are in the form of an O/W or W/O emulsion or microemulsion.

The term "encapsulated sunscreen agent" refers to a sunscreen agent which is coated by a suitable capsule wall material to form microcapsules of a core shell or a matrix type, preferably of a core-shell type. The sunscreen agent is either in the form of a discrete liquid or a discrete solid. Also mixtures of sunscreen agents may be employed as well as sunscreen agents diluted or solubilized in a suitable solvent, preferably a cosmetically acceptable solvent. The microcapsules may be prepared by various polymerization techniques known in the art such as the sol-gel method, an in-situ-polymerisation method or an emulsion polymerization method, such as an ex-situ emulsion polymerization method. Microcapsules obtainable by the sol-gel method or an ex-situ emulsion polymerization process are preferred according to the present invention.

The microcapsules contain usually more than 10 wt.-% of the sunscreen agent, and preferably 10 to 95 wt.-%, e.g. 50 to 95 wt.% of the microcapsules consist of the sunscreen agent, more preferably 70 to 95 wt.-%.

The above percentages are weight percentages based on the total weight of the microcapsules.

"Percentages" and "parts" used in the present invention are generally weight percent or parts by weight, if nothing else is stated or obvious.

The compositions of the present invention contain preferably 1 to 50 wt.-% of the microcapsules containing the sunscreen agent, more preferably 1 (or 3, or 5) to 30 wt.-%, more preferably 3 to 20 wt.-%, such as 5 to 20 wt.-%.

The present invention also provides the encapsulated cinnamic ester derivatives of formula I 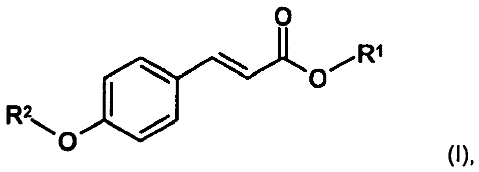 as defined in claim 14, which are the most preferred first sunscreen agents in the compositions of the present invention.

These encapsulated cinnamic ester derivatives according to the present invention are particularly useful for providing the (topical) compositions of the invention, in particular pharmaceutical and cosmetic compositions, however, they can, of course, also be incorporated into other compositions or used for other purposes as known in the art. Thus, while the encapsulated sunscreen agents of the present invention are particularly suitable for the protection of human skin or hair from damaging UV radiation, they can also be used for the protection of UV-sensitive plastic materials, medicinal products and other objects.

The encapsulated sunscreen agents of the present invention can be in the form of an aqueous dispersion or a suspension containing about 1 to 60% solids, preferable 20-40% solids as sphere particles with the required D(v, 0.5).

The pH of the suspension can be in the range of 3 to 10.

In another embodiment the encapsulated sunscreen agent is in the form of a fine powder consisting of sphere particles with the required D(v, 0.5).

By providing a composition according to the invention with the first encapsulated sunscreen agent and the second different sunscreen agent, the photostability of the sunscreen agent which is encapsulated can be enhanced. Therefore, the present invention also provides a method for enhancing the photostability of a cinnamic ester derivative which is in the form of an encapsulated cinnamic acid derivative as defined above, wherein the cinnamic ester derivative is incorporated into a topical composition containing a further sunscreen agent, preferably selected from selected from PABA, camphor benzalkonium methosulfate, homosalate, benzophenone-3, phenylbenzimidazole sulfonic acid (e.g. PARSOL^{®} HS), terephthalidene dicamphor sulfonic acid (e.g Mexoryl SX), butyl methoxydibenzoylmethane (e.g. PARSOL^{®} 1789), benzylidene camphor sulfonic acid (e.g. Mexoryl SL), octocrylene (e.g. PARSOL^{®} 340), polyacrylamidomethyl benzylidene camphor, PEG-25 PABA, isoamyl p-methoxycinnamate, ethylhexyl triazone (e.g. UVINUL^{®} T150), drometrizole trisiloxane (Mexoryl XL), diethylhexyl butamido triazine (e.g. Uvasorb^{®} HEB), 4-methylbenzylidene camphor (e.g. PARSOL^{®} 5000), 3-benzylidene camphor, ethylhexyl salicylate, ethylhexyl dimethyl PABA, benzophenone-4, methylene-bis-benzotriazolyl tetramethylbutylphenol (e.g. Tinosorb M), disodium phenyl dibenzimidazol tetrasulfonate (Neo Heliopan^{®} AP), bis-ethylhexyloxyphenol methoxyphenyl triazine (Tinosorb^{®} S), polysilicone-15 (PARSOL^{®} SLX), diethylamino hydroxybenzoyl hexyl benzoate (UVINUL^{®} A plus), microfine titanium dioxide (e.g. PARSOL^{®} TX) and microfine zinc oxide. DEA-methoxycinnamate, diisopropyl methyl cinnamate, 1-(3,4-dimethoxyphenyl)-4,4-dimethyl-1,3-pentanedione, ethyl diisopropylcinnamate, 2-ethylhexyl dimethoxybenzylidene dioxoimidazolidine propionate, ethyl PABA, menthyl anthranilate, potassium phenylbenzimidazole sulfonate, sodium phenylbenzimidazole sulfonate, TEA-salicylate, 2,2-(1,4-phenylene)bis-(1H-benzimidazole-4,6-disulfonic acid and, 2-(4-diethylamino-2-hydroxy-benzoyl)-benzoic acid hexylester.

Preferably, the second sunscreen is selected from phenylbenzimidazole sulfonic acid (e.g. PARSOL^{®} HS), butyl methoxydibenzoylmethane (e.g. PARSOL^{®} 1789), octocrylene (e.g. PARSOL^{®} 340), ethylhexyl triazone (e.g. UVINUL^{®} T150), diethylhexyl butamido triazine (e.g. Uvasorb^{®} HEB), methylene-bis-benzotriazolyl tetramethylbutylphenol (e.g. Tinosorb M), disodium phenyl dibenzimidazol tetrasulfonate (Neo Heliopan^{®} AP), bis-ethylhexyloxyphenol methoxyphenyl triazine (Tinosorb^{®} S), polysilicone-15 (PARSOL^{®} SLX), diethylamino hydroxybenzoyl hexyl benzoate (UVINUL^{®} A plus) or microfine titanium dioxide.

Most preferably, the second sunscreen agent is butyl methoxydibenzoylmethane, ethylhexyl triazone (e.g. UVINUL^{®} T150), methylene-bis-benzotriazolyl tetramethylbutylphenol (e.g. Tinosorb M), bis-ethylhexyloxyphenol methoxyphenyl triazine (Tinosorb^{®} S) or polysilicone-15 (PARSOL^{®} SLX).

By providing a composition according to the invention wherein the first encapsulated sunscreen agent is a cinnamate derivative and the second non-encapsulated sunscreen agent is a dibenzoylmethane derivative, the photostability of both sunscreen agents can be significantly enhanced by avoiding their cross reactivity. Therefore, the present invention also provides a method for enhancing the photostability of a dibenzoylmethane, preferably butyl methoxydibenzoylmethane in topical cosmetic compositions comprising a cinnamic ester derivative which is in the form of an encapsulated cinnamic acid derivative as defined above. Preferably the topical composition comprises at least one additional third sunscreen agent which is suitable for further photostabilising the dibenzoylmethane derivative such as benzophenones-3, benzophenones-4, polysilicones-15 and/ or octocrylene, preferably polysilicones-15 and/ or octocrylene. Thus, the invention also relates to topical cosmetic compositions comprising an encapsulated cinnamate derivative as defined above and a dibenzoylmethane derivative, preferably butyl methoxydibenzoylmethane.

Additionally, the invention relates to photostable compositions comprising an encapsulated cinnamate derivative as defined above, a dibenzoylmethane derivative, preferably butyl methoxydibenzoylmethane and at least a third sunscreen agent which is suitable for further photostabilising the dibenzoylmethane derivative such as benzophenones-3, benzophenones-4, polysilicones-15 and/ or octocrylene, preferably polysilicones-15 and/ or octocrylene. Optionally further sunscreen agents may be present.

If the third sunscreen agent is octocrylene, the amount used is not critical, preferably it is used in a ratio to butyl methoxydibenzoylmethane of being less than 0.8 as e.g. disclosed in EP 0780119 B1, claim 1.

If the third sunscreen agent is polysilicones-15, the amount used is not critical. Preferably it is used in an amount of at least 0.2 wt% in particular in an amount of 0.5 to 10 wt.%, more particular in an amount of 1 to 5 wt.% based on the total weight of the formulation.

The encapsulated sunscreen agents in the form of microcapsules having an average particle size D(v, 0.5) in the range from 3 µm to 8 µm (preferably 3 to 6, 3 to 5 and most preferably 3 to 4 µm) can principally be prepared according to the methods known in the art, however, the polymerization methods must be adjusted to provide microcapsules having the required average particle size.

Preferably the ratio (weight by weight) of the encapsulating agent, in particular the tetraalkoxysilane, such as TEOS to the sunscreen agent, such as the cinnamie ester derivative is in the range of 6:1 to 1:13, in particular in the range of 2:1 to 1:10, such as in the range of 1:1 to 1:10 or 1,2:1 to 1:7,3 or 1:1,37 to 1:6,1.

The sunscreen agent is encapsulated by a polymer obtained from a water-reactive, silicon-based precursor namely tetraalkoxysilane, e.g. tetraethoxysilane and by using an emulsifying step to obtain microcapsules. It can particularly be referred to the preparation method which is disclosed in WO 2003/066209 which is included herein by reference insofar as it refers to the encapsulation method. According to the method disclosed in this document, the polymer precursor is added to an emulsion of the functional molecule thus allowing the polymer precursor to polymerize around preformed particles of the functional molecule. The encapsulation technology comprises the steps of:
(a) Preparing an aqueous emulsion of the sunscreen agent which emulsion has a positive zeta potential;
(b) Adding a water-reactive silicon compound, e.g., a tetraalkoxysilane to said emulsion; and
(c) Allowing the tetraalkoxysilane to polymerize at the interface of the emulsified droplets of the sunscreen agent to form microcapsules wherein the sunscreen agent is surrounded by a shell of silicon-based polymer.

The positive zeta potential of the emulsion of step (a) can be achieved by the presence of a cationic surfactant. The polymerization can proceed under acidic, neutral or basic conditions. This process exemplifies an ex-situ emulsion polymerization process of tetraethoxysiloxane (TEOS). Preferably the ratio (weight by weight) of tetraalkoxysilane such as TEOS to the sunscreen agent such as the cinnamic acid derivative is in the range of 6/1 to 1/13, in particular from 1/1 to 1/10.

Another preferred process example exemplifying an ex-situ polymerization process for preparing the encapsulated sunscreen agents according to the invention includes
1) Mixing an oil phase and an aqueous solution of a cationic surfactant to form an oil in water emulsion
2) adding a water reactive silicone compound comprising a tetraalkoxysilane to the oil in water emulsion,
3) polymerizing the tetraalkoxysilane at the oil/ water interface of the emulsion to form a microcapsule having a core containing the oil and the shell,
wherein the weight % of cationic surfactant to the oil phase of step 1) ranges from 0.1% to 0.3%. Preferably the tetraalkoxysilane is tetraethoxysilane (TEOS) and the cationic surfactant is cetyl trimethyl ammonium chloride. The ratio (weight by weight) of tetraalkoxysilane to the oil phase such as the cinnamic acid derivative is in the range of 6/1 to 1/13, alternatively from 1.2/1 to 1/7.3, alternatively from 1/1.37 to 1/6.1.

Another preferred process for preparing the encapsulated sunscreen agents according to the invention or enclosed in the topical compositions according to the invention is disclosed in US-A 6,303,140 which is also included herein by reference regarding the production process. According to this document polymerization takes place in a preformed emulsion of a functional molecule and a polymer precursor. The process disclosed in this document generally comprises the following steps:
(a) Preparing an aqueous emulsion of a non-aqueous water-insoluble solution containing sol-gel precursors, and a functional molecule, in the presence of a surfactant;
(b) Stirring the emulsion of step (a) with an added acidic, neutral or basic aqueous solution to form a suspension of sol-gel microcapsules.

The sol-gel precursor may be tetraethoxysilane. The surfactant may be a cationic surfactant. This process exemplifies a Sol-Gel process of TEOS.

For details of the process it can be referred e.g. to example 1 of US-A 6,303,149.

The particle size can be adjusted in the above process by using an appropriate homogenizer pressure, however the suitable homogenizer pressure has to be adjusted based on the model used. In general higher homogenizer pressure leads to smaller particle sizes. Useful homogenizers are any emulsification devices known in the art. The homogenizer pressure can vary depending on the homogenizer used. An appropriate homogenzer pressure for obtaining a particle size with a D(v, 0.5) in the range from 3 µm to 8 µm is normally in the range of 10-200bars, preferably between 30 and 100 bars.

Another preferred process for preparing the encapsulated sunscreen agents according to the invention or enclosed in the topical compositions according to the invention is disclosed in WO 2005/009604 which is also included herein by reference regarding the production process and which exemplifies an in-situ polymerization process of TEOS.

If desired, additional UV-A and UV-B screening agents may be added to the cosmetic and/or dermatological compositions of the present invention. The combination of different UV filters may also show synergistic effects.

While the encapsulated sunscreens can be used alone or in combination with other compounds which absorb in the UV range, although at least an effective amount of the encapsulated sunscreens should be present in the sunscreen compositions. The term "effective amount of the encapsulated sunscreens" means generally at least 0.2% by weight of the light screening agent (without encapsulating polymer) based on the total weight of the sunscreen composition.

The total amount of UV screening agents, i.e. of the encapsulated sunscreens (without encapsulation polymer) and, if desired, of additional UV-A/B screening agents, is not narrowly critical. Such amounts may vary from 0.2% by weight and higher, suitably between about 0.5 and about 20%, preferably between about 0.5 and about 12% by weight of the total amount of the composition.

Suitable UV-B screening agents which may be contained in the compositions of the present invention are, e.g., the following organic and inorganic compounds:
- Acrylates, such as 2-ethylhexyl-2-cyano-3,3-diphenylacrylate (octocrylene, PARSOL® 340), ethyl 2-cyano-3,3-diphenylacrylate and the like;
- camphor derivatives such as 4-methyl benzylidene camphor (PARSOL® 5000), 3-benzylidene camphor, camphor benzalkonium methosulfate, polyacrylamidomethyl benzylidene camphor, sulfo benzylidene camphor, sulfomethyl benzylidene camphor, therephthalidene dicamphor sulfonic acid and the like;

- cinnamate derivatives such as octyl methoxycinnamate (PARSOL® MCX), ethoxyethyl methoxycinnamate, diethanolamine methoxycinnamate (PARSOL® Hydro), isoamyl methoxycinnamate and the like, as well as cinnamic acid derivatives bound to siloxanes;
- p-aminobenzoic acid derivatives, such as p-aminobenzoic acid, 2-ethylhexyl p-dimethylaminobenzoate, N-oxypropylenated ethyl p-aminobenzoate, glyceryl p-aminobenzoate;
- benzophenones, such as benzophenone-3, benzophenone-4, 2,2',4,4'-tetrahydroxybenzophenone, 2,2'-dihydroxy-4,4'-dimethoxybenzophenone and the like;
- esters of benzalmalonic acid such as di-(2-ethylhexyl)-4-methoxybenzalmalonate;
- esters of 2-(4-ethoxy-anilinomethylene)-propanedioic acid, such as 2-(4-ethoxy-anilinomethylene)-propanedioic acid diethyl ester (EP-A2 0 895 776);
- organosiloxane compounds containing benzmalonate groups as described in the European Patent Publications EP-B1 0 358 584, EP-B1 0 538 431 and EP-A1 0 709 080 or other organosiloxanes disclosed in the prior art documents discussed in the introductory part of the present application;
- drometrizole trisiloxane (Mexoryl XL);
- pigments such as microparticulated TiO₂, and the like, the term "microparticulated" referring to a particle size from about 5 nm to about 200 nm, particularly from about 15 nm to about 100 nm. The TiO₂ particles may also be coated by metal oxides such as aluminum or zirconium oxide, or by organic coatings such as polyols, methicone, aluminum stearate, alkyl silane and the like, well known in the art;
- imidazole derivatives such as, 2-phenyl benzimidazole sulfonic acid and its salts (PARSOL®HS), e.g., alkali salts such as sodium or potassium salts, ammonium salts, morpholine salts, salts of primary, sec. and tert. amines like monoethanolamine salts, diethanolamine salts and the like;
- salicylate derivatives such as isopropylbenzyl salicylate, benzyl salicylate, butyl salicylate, octyl salicylate (NEO HELIOPAN OS), isooctyl salicylate or homomenthyl salicylate (homosalate, HELIOPAN) and the like;
- triazone derivatives such as octyl triazone (UVINUL T-150), dioctyl butamido triazone (UVASORB HEB) and the like.
- Suitable conventional UV-A screening agents which may be contained in the compositions of the present invention are the following organic and inorganic compounds:
- Dibenzoylmethane derivatives such as 4-tert.-butyl-4'-methoxydibenzoyl-methane (PARSOL^{®} 1789), dimethoxydibenzoylmethane, isopropyldibenzoylmethane and the like;
- benzotriazole derivatives such as 2,2'-methylene-bis-[6-(2H-benzotriazole-2-yl)-4-(1,1,3,3,-tetramethylbutyl)-phenol] (TINOSORB M) and the like;
- phenylene-1,4-bis-benzimidazolsulfonic acids or their salts such as 2,2-(1,4-phenylene)-bis-(1H-benzimidazol-4,6-disulfonic acid) (Neoheliopan AP);
- amino substituted hydroxybenzophenones as described in European patent publication EP 1 046 391 such as 2-(4-diethylamino-2-hydroxy-benzoyl)-benzoic acid hexylester (INCI diethylamino hydroxybenzoyl hexyl benzoate, UVINUL^{®} A plus);
- pigments such as microparticulated ZnO and the like. The term "microparticulated" refers to a particle size from about 5 nm to about 200 nm, particularly from about 15 nm to about 100 nm. The ZnO particles may also be coated by metal oxides such as, e.g., aluminum or zirconium oxides or by organic coatings such as e.g. polyols, methicone, aluminum stearate, alkyl silane. Such coatings are well known in the art.

Because dibenzoylmethane derivatives are photolabile UV-A screening agents, it may be desirable to photostabilize them. Thus, the term "conventional UV-A screening agent" also refers to diberizoylmethane derivatives such as e.g. PARSOL^{®} 1789 stabilized by, e.g.,
- 3,3-diphenylacrylate derivatives as described in EP-B1 0 514 491 and EP-A1 0 780 119;
- benzylidene camphor derivatives as described in USP-5,605,680;
- organosiloxanes containing benzmalonate groups as described in EP-B1 0 358 584, EP-B1 538 431 and EP-A1 0 709 080 or other organosiloxanes disclosed in the prior art documents discussed in the introductory part of the present application.

The compositions of the invention can also contain usual cosmetic adjuvants and additives, such as preservatives/antioxidants, fatty substances/oils, water, organic solvents, silicones, thickeners, softeners, emulsifiers, additional sunscreens, antifoaming agents, moisturizers, fragrances, surfactants, fillers, sequestering agents, anionic, cationic, nonionic or amphoteric polymers or mixtures thereof, propellants, acidifying or basifying agents, dyes, colorants, pigments or nanopigments, in particular those suitable for providing an additional photoprotective effect by physically blocking out ultraviolet radiation, or any other ingredients usually formulated into cosmetics, in particular for the production of sunscreen/antisun compositions. The necessary amounts of the cosmetic and dermatological adjuvants and additives can, based on the desired product, easily be chosen by the skilled person.

Particularly preferred antioxidants are those chosen from the group consisting of amino acids (e.g. glycine, histidine, tyrosine, tryptophan) and their derivatives, imidazole (e.g. urocanic acid) and derivatives, peptides such as D,L-carnosine, D-carnosine, L-carnosine and derivatives (e.g. anserine), carotenoids, carotenes (e.g. β-carotene, γ-carotene, lycopene) and derivatives, chlorogenic acid and derivatives, liponic acid and derivatives (e.g. dihydroliponic acid), aurothioglucose, propylthiouracil and other thiols (e.g. thioredoxin, glutathione, cysteine, cystine, cystamine and its glycosyl-, N-acetyl-, methyl-, ethyl-, propyl-, amyl-, butyl- and lauryl-, palmitoyl-, oleyl-, γ-linoleyl-, cholesteryl- and glycerylester) and the salts thereof, dilaurylthiodipropionate, distearylthiodipropionate, thiodipropionic acid and its derivatives (esters, ethers, peptides, lipids, nucleotides, nucleosides and salts) as well as sulfoximine compounds (such as buthioninesulfoximine, homocysteinesulfoximine, buthioninesulfone, penta-, hexa-, heptathioninesulfoximine) in very low compatible doses (e.g. from pmol to µmol/kg), additional (metal)-chelators (such as α-hydroxy fatty acids, palmic acid, phytinic acid, lactoferrin), α -hydroxyacids (such as citric acid, lactic acid, malic acid), huminic acid, gallic acid, gallic extracts, bilirubin, biliverdin, EDTA, EGTA and its derivatives, unsaturated fatty acids and their derivatives (such as γ-linoleic acid, linolic acid, oleic acid), folic acid and its derivatives, ubiquinone and ubiquinol and their derivatives, vitamin C and derivatives (such as ascorbylpalmitate, Mg-ascorbylphosphate, Na- ascorbylphosphate, ascorbylacetate), tocopherol and derivates (such as vitamin E acetate), vitamin A and derivatives (vitamin A palmitate) as well as coniferylbenzoate, rutinic acid and derivatives, α-glycosylrutin, ferulic acid, furfurylideneglucitol, carnosine, butylhydroxytoluene, butylhydroxyanisole, trihydroxybutyrophenone, urea and its derivatives; mannose and derivatives, zinc and derivatives (e.g. ZnO; ZnSO₄), selen and derivatives (e.g. selenomethionine), stilbenes and derivatives (such as stilbeneoxide, trans-stilbeneoxide) and suitable derivatives (salts, esters, ethers, sugars, nucleotides, nucleosides, peptides and lipids) of the named active ingredients.

The amount of the abovementioned preservatives and/ or antioxidants (one or more compounds) in the preparations is preferably from 0.001 to 30% by weight, particularly preferably from 0.05 to 20% by weight, in particular from 1 to 10% by weight, based on the total weight of the preparation.

If vitamin E and/or derivatives thereof are used as the antioxidant(s), it is advantageous to choose their particular concentration from the range 0.001 to 10% by weight, based on the total weight of the formulation.

If vitamin A and/or derivatives thereof, or carotenoids are the antioxidant(s), it is advantageous to choose their particular concentration from the range 0.001 to 10% by weight, based on the total weight of the formulation.

The compositions according to the present invention may also contain emulsifiers. An emulsifier enables two or more immiscible liquids to be combined homogeneously, while increasing the viscosity of the composition. Moreover, the emulsifier acts to stabilize the composition.

Emulsifiers that may be used according to the present invention, to form O/W, W/O and/or O/W/O formulations, include sorbitan oleate, sorbitan sesquioleate, sorbitan isostearate, sorbitan trioleate, polyglyceryl-3-diisostearate, polyglycerol esters of oleic/isostearic acid, polyglyceryl-6 hexaricinolate, polyglyceryl-4-oleate, polygylceryl-4 oleate/PEG-8 propylene glycol cocoate, oleamide DEA, TEA myristate, TEA stearate, magnesium stearate, sodium stearate, potassium laurate, potassium ricinoleate, sodium cocoate, sodium tallowate, potassium castorate, sodium oleate, and mixtures thereof. Further suitable emulsifiers are phosphate esters and salts thereof such as cetyl phosphate, DEA cetyl phosphate, potassium cetyl phosphate, sodium glyceryl oleate phosphate, hydrogenated vegetable glyceride phosphates and mixtures thereof. Furthermore, one or more synthetic polymers may be used as emulsifiers. For example, PVP eicosaene copolymer, acrylates/C10-C30 alkyl acrylate crosspolymer, acrylates/steareth methacrylate copolymer, PEG-22/dodecyl glycol copolymer, PEG-45/dodecyl glycol copolymer, and mixtures thereof. Preferred emulsifiers are PVP eicosaene copolymer, acrylates/C10-C30 alkyl acrylate crosspolymer, PEG-20 sorbitan isostearate, sorbitan isostearate, and mixtures thereof.

The emulsifier may be present in a total amount varying from about 0.01 wt.-% to about 15 wt.-%, preferably from about 0.1 wt.-% to about 3 wt.-%, of the total weight of the composition.

The fatty/oily phase is advantageously chosen from:
- mineral oils and mineral waxes;
- oils such as triglycerides of caprinic acid or caprylic acid, preferably castor oil;
- natural or synthetic oils, preferably esters of carbonic acids or fatty acids with alcohols, e.g., such as isopropanol, propylene glycol or glycerin;
- alkylbenzoates and
- silicone oils such as dimethylpolysiloxane, diethylpolysiloxane, diphenylpolysiloxane and mixtures thereof.

Fatty substances which can be incorporated into the oily phase of the compositions according to the invention are advantageously chosen from esters of saturated and/or unsaturated, straight or branched chain alkyl carboxylic acids with 3 to 30 carbon atoms, and saturated and/or unsaturated, straight and/or branched chain alcohols with 3 to 30 carbon atoms, as well as esters of aromatic carboxylic acids and of saturated and/or unsaturated, straight or branched chain alcohols of 3 to 30 carbon atoms. Such esters can advantageously be selected from octylpalmitate, octylcocoate, octylisostearate, octyldodecylmyristate, cetylisononanoate, isopropylmyristate, isopropylpalmitate, isopropylstearate, isopropyloleate, n-butylstearate, n-hexyllaurate, n-decyloleate, isooctylstearate, isononylstearate, isononylisononanoate, 2- ethyl hexylpalmitate, 2-ethylhexyllaurate, 2-hexyldecylstearate, 2-octyldodecylpalmitate, stearylheptanoate, oleyloleate, oleylerucate, erucyloleate, erucylerucate, tridecylstearate, tridecyltrimellitate, as well as from synthetic, half-synthetic and natural mixtures of such esters such as jojoba oil.

Other fatty components suitable for use in the compositions according to the present invention include polar oils such as lecithins and fatty acid triglycerides, namely triglycerinic esters of saturated and/or unsaturated, straight or branched chain carbonic acids with 8 to 24 carbon atoms, preferably of 12 to 18 carbon atoms whereas the fatty acid triglycerides are preferably chosen from synthetic, half synthetic and natural oils (e.g. cocoglyceride, olive oil, sun flower oil, soybean oil, peanut oil, rape oil, almond oil, palm oil, coconut oil, castor oil, hydrogenated castor oil, wheat oil, grape oil and others); apolar oils such as linear and/ or branched chain hydrocarbons and waxes, e.g., mineral oils, vaseline (petrolatum); paraffins, squalane and squalene, polyolefins (favored are polydecenes), hydrogenated polyisobutenes and isohexadecanes; dialkyl ethers such as dicaprylylether; linear or cyclic silicone oils such as cyclomethicone, octamethylcyclotetrasiloxane, cetyldimethicone, hexamethylcyclotrisiloxane, polydimethylsiloxane, poly-(methylphenylsiloxane) and mixtures thereof.

Other fatty components which can advantageously be incorporated into the compositions of the present invention are isoeikosane; neopentylglycoldiheptanoate; propylene glycoldicaprylate/-dicaprate; caprylic-/capric-/diglycerylsuccinate; butylene glycol caprylat/caprat; C12-13 alkyllactate; di-C12-13 alkyltartrate; triisostearin; dipentaerythrityl hexacaprylate/hexacaprate; propylene glycolmonoisostearate; tricapryline; dimethylisosorbid. Particularly preferred is the use of mixtures of C12-15 alkylbenzoate and 2-ethylhexylisostearate, mixtures of C12-15 alkylbenzoate and isotridecylisononanoate as well as mixtures of C12-15 alkylbenzoate, 2-ethylhexylisostearate and isotridecylisononanoate.

The oily phase of the compositions according to the present invention can also contain natural vegetable or animal waxes such as bee wax, china wax, bumblebee wax and other waxes of insects as well as shea butter.

The compositions according to the present invention may additionally contain one or more emollients. An emollient provides a softening or soothing effect on the skin surface and is generally considered safe for topical use. Emollients also help control the rate of evaporation and the tackiness of the composition. Preferred emollients include mineral oil, lanolin oil, coconut oil, cocoa butter, olive oil, aloe extracts, jojoba oil, castor oil, fatty acids such as oleic and stearic acid, fatty alcohols such as cetyl and hexadecyl alcohol diisopropyl adipate, benzoic and hydroxybenzoic acid esters of C₉-C₁₅ alcohols, isononyl iso-nonanoate, C₁₅-C₅₀ alkanes, mineral oil, silicones such as dimethyl polysiloxane, ethers such as polyoxypropylene butyl ethers and polyoxypropylene cetyl ethers, and C₂-C₁₅ alkyl benzoates, and mixtures thereof. The most preferred emollients are hydroxybenzoate esters, aloe vera, C₁₂₋₁₅ alkyl benzoates, and mixtures thereof.

The emollient is present in an amount varying from about 1 wt.-% to about 20 wt.-%, preferably from about 2 wt.-% to about 15 wt.-%, and most preferably from about 4 wt.-% to about 10 wt.-% of the total weight of the composition.

The aqueous phase of the formulations of the present invention can contain the usual cosmetic additives such as alcohols, especially lower alcohols, preferably ethanol and/ or isopropanol, low alkyl diols or polyols and their ethers, preferably propylene glycol, glycerin, ethylene glycol, ethylene glycolmonoethyl- or -monobutyl ether, propylene glycolmonomethyl-, -monoethyl- or -monobutyl ether, diethylene glycolmonomethyl- or -monoethyl ether and analogue products, polymers, foam stabilizers; electrolytes and, especially, one or more thickeners.

Thickeners that may be used in formulations of the present invention include the family of silicium dioxide, magnesium and/or aluminum silicates, polysaccharides and their derivatives such as hyaluronic acid, xanthan gum, hydroxypropyl cellulose, acrylate copolymers, preferably a polyacrylate of the family of carbopoles, such as carbopoles of type 980, 981, 1382, 2984, 5984.

Moisturizing agents, such as humectants, may be incorporated into the compositions according to the present invention to reduce the trans-epidermal water loss (TEWL) of the horny layer of the skin. Suitable humectants include glycerin, lactic acid, pyrrolidone carbonic acid, urea, polyethylene glycol, polypropylene glycol, sorbitol, PEG-4, and mixtures thereof. Additional suitable moisturizers are polymeric moisturizers of the family of water soluble and/or with water gelating polysaccharides such as hyaluronic acid, chitosan and/or fucose rich polysaccharides available, e.g., as Fucogel®1000 (CAS-Nr. 178463-23-5) from SOLABIA S. The moisturizing agent is optionally present in an amount varying from about 0.5 wt.-% to about 8 wt.-%, preferably from about 1 wt.-% to about 5 wt.-% of the total weight of the composition.

Suitable neutralizing agents which may be included in the composition of the present invention to neutralize components such as e.g. an emulsifier or a foam builder/stabilizer include but are not limited to alkali hydroxides such as a sodium and potassium hydroxide; organic bases such as diethanolamine (DEA), triethanolamine (TEA), aminomethyl propanol, trisodium ethylene diaminetetraacetic acid and mixtures thereof; basic amino acids such as arginine and lysine and any combination of any of the foregoing. The neutralizing agent may be present in an amount of about 0.01 wt.-% to about 8 wt.-% in the compositions of the present invention, preferably, 1 wt.% to about 5 wt.-%.

The addition of electrolytes into the composition of the present invention may be necessary to change the behavior of a hydrophobic emulsifier. Thus the emulsions/microemulsions of this invention may preferably contain electrolytes of one or several salts including anions such as a chloride, a sulfate, a carbonate, a borate or an aluminate, without being limited thereto. Other suitable electrolytes may be on the bases of organic anions such as, but not limited to, lactate, acetate, benzoate, propionate, tartrate and citrate. As cations preferably ammonia, alkylammonia, alkali- or alkaline earth metals, magnesium-, iron- or zinc-ions are selected. Especially preferred salts are potassium and sodium chloride, magnesium sulfate, zinc sulfate and mixtures thereof. Electrolytes are present in an amount of about 0.01 wt.-% to about 8 wt.-% in the compositions of the present invention.

The cosmetic compositions of the invention are useful as compositions for photoprotecting the human epidermis or hair against the damaging effect of UV irradiation, as antisun/ sunscreen composition or as makeup product. Such compositions can, in particular, be provided in the form of a lotion, a thickened lotion, a gel, a cream, a milk, an ointment, a powder or a solid tube stick and may optionally be packaged as an aerosol and may be provided in the form of a mousse, foam or a spray. When the cosmetic composition according to the invention is provided for protecting the human epidermis against UV radiation or as antisun/ sunscreen composition, it may be in the form of a suspension or dispersion in solvents or fatty substances, or alternatively in the form of an emulsion or microemulsion (in particular of O/W or W/O type, O/W/O or W/O/W-type), such as a cream or a milk, a vesicular dispersion, in the form of an ointment, a gel, a solid tube stick or an aerosol mousse. The emulsions can also contain anionic, nonionic, cationic or amphoteric surfactants.

When the cosmetic composition according to the invention is used for protecting the hair, it may be in the form of a shampoo, a lotion, a gel or a rinse out composition, to be applied before or after shampooing, before or after dyeing or bleaching, before, during or after permanent-waving or hair straightening operation, a styling or treatment lotion or a gel, a blow-drying or hairsetting lotion or gel, a hair lacquer, or a composition for permanent-waving, straightening, dyeing or bleaching the hair.

To protect human hair against UV rays, the encapsulated sunscreens can be incorporated into shampoos, lotions, gels, hairsprays, aerosol foam creams or emulsions in concentrations of from 0.1 to 10% by weight, preferably from 1 to 7% by weight. The respective formulations can be used, inter alia, for washing, coloring and for styling the hair. When the cosmetic composition according to the invention is used as makeup product for eyelashes, the eyebrows, the skin or the hair, such as an epidermal treatment cream, a foundation, a tube of lipstick, an eyeshade, a face powder, an eyeliner, a mascara or a coloring gel, it may be solid or pasty, anhydrous or in aqueous form, such as O/W or W/O emulsion, suspension or gel.

The present invention also features formulating the encapsulated sunscreen agents as agents for screening out UV radiation, in particular for controlling the color of human skin.

The encapsulated sunscreen agents show an excellent liposolubilities and can thus be incorporated in high concentrations into cosmetic formulations leading to a high protection factor of the final compositions. Additionally they are homogeneously distributed in the cosmetic formulation containing at least a fatty phase and a cosmetically accepted organic solvent which leads, applied on the skin/or hair, to the formation of a protective film which protects effectively the skin and/or hair against the deleterious effects of UV-radiation.

Thus, it is another object of the present invention to use the compounds and compositions of the invention for protecting the skin and/ or hair against UV radiation, in particular solar radiation, comprising topically applying an effective amount of a cosmetic composition containing the encapsulated sunscreen agents.

Finally, according to another embodiment of the invention, an encapsulated sunscreen agent or compositions of this invention can be used as protecting agents against UV radiation for plastics and other UV sensitive materials and products.

The following examples are provided to further illustrate the processes and compositions of the present invention. These examples are illustrative only and are not intended to limit the scope of the invention in any way.

All measurements of particle size referred to in this specification are made by laser diffraction technique using a "Mastersizer 2000" of Malvern Instruments Ltd., UK, and further information on the above particle sizes can e.g. be found in "Basic principles of particle size analytics", Dr. Alan Rawle, Malvern Instruments Limited, Enigma Business Part, Grovewood Road, Malvern, Worcestershire, WR14 1XZ, UK and the "Manual of Malvern particle size analyzer". Particular reference is made to the user manual manual number MAN 0096, Issue 1.0, Nov. 1994. All particle sizes indicated in the present application are mean particle sizes according to D(v, 0.5) and are measured with a Malvern Mastersizer, if nothing else is stated or obvious.

### Sample preparation

### Example 1

11 g EHMC are dissolved in 33 g TEOS. The organic phase is emulsified in 200 g of aqueous solution containing 1% cetyltrimethyl ammonium chloride (CTAC) under high shear forces using Ultra-Turrax T-25 basic with S25 KR-18G dispersing tool (IKA) at 17500 rpm. The emulsion is then poured into a beaker containing 200 g NaOH aqueous solution at pH 11.3. The solution is stirred at 400 rpm while the emulsion is added, then the stirring rate lowered to 200 rpm. The emulsion is stirred at room temperature for 24 hours, followed by stirring at 50°C for 3 hours. The resulting suspension contains microcapsules with a D(v, 0.5) of 1.4 µm.

### Example 2

35 g EHMC is emulsified in 100 g of water containing 0.2 g Volpo L3 and 0.3 g of cetyltrimethyl ammonium chloride (CTAC) with an APV-1000 (APV Homogenisers AS) at 60 bar. 10 g of TEOS is added to the emulsion while stirring to form a coarse emulsion of microcapsules. The resulting suspension contains microcapsules with a D(v, 0.5) of 3.5 µm.

### Example 3

35 g EHMC is emulsified in 100g of water containing 0.2 g Volpo L3 (INCI C12-13 pareth-3) and 0.3 g of cetyltrimethyl ammonium chloride (CTAC) with an APV-1000 (APV Homogenisers AS) at 70 bar. 15 g of TEOS is added to the emulsion while stirring to form a coarse emulsion of microcapsules. The resulting suspension contains microcapsules with a D(v, 0.5) of 3.8 µm.

### Example 4

35 g EHMC are emulsified in 100 g of water containing 0.2 g Volpo L3 and 0.3 g of cetyltrimethyl ammonium chloride (CTAC) with an APV-1000 (APV Homogenisers AS) at 80 bar. 13 g of TEOS is added to the emulsion while stirring to form a coarse emulsion of microcapsules. The resulting suspension contains microcapsules with a D(v, 0.5) of 3.05 µm.

### Example 5

35 g EHMC are emulsified in 100 g of water containing 0.2 g Volpo L3 and 0.3 g of cetyltrimethyl ammonium chloride (CTAC) with an APV-1000 (APV Homogenisers AS) at 40 bar. 10 g of TEOS is added to the emulsion while stirring to form a coarse emulsion of microcapsules. The resulting suspension contains microcapsules with a D(v, 0.5) of 4.0 µm.

### Example 6

30 g EHMC is dissolved in 2.5 g TEOS. The organic phase is emulsified in 17 g of aqueous solution containing 1% cetyltrimethyl ammonium chloride (CTAC) under high shear forces using Ultra Homogenisers AS at 500 bars. The emulsion is then poured into a beaker containing 25 g of an aqueous solution having a pH of 3.8. The solution is stirred at 400 rpm while the emulsion is added, then the stirring rate lowered to 60 rpm. The emulsion is stirred at room temperature for 24 hours, followed by stirring at 50°C for 3 hours. The resulting suspension contains microcapsules with a D(v, 0.5) of 1.7 µm.

### Example 7

350 g EHMC is emulsified in 540.9 g water containing 1.4g Pareth-3 nonionic polyethylene glycol lauryl ether surfactant and 0.9 g cetyltrimethyl ammonium chloride (CTAC). The coarse emulsion is passed once through a 'Rannie Mini Lab 8.30 H' homogenizer operating at 100 bars. 10.46% TEOS is added to the emulsion while stirring to form a coarse emulsion of microcapsules with a D(v, 0.5) of 3.55 µm.

### Example 8

350 g EHMC is emulsified in 540.9 g water containing 1.4g Pareth-3 nonionic polyethylene glycol lauryl ether surfactant and 0.9 g cetyltrimethyl ammonium chloride (CTAC). The coarse emulsion is passed once through a 'APV model 1000' homogenizer operating at 40 bars. 10.46% TEOS is added to the emulsion while stirring to form a coarse emulsion of microcapsules with a D(v, 0.5) of 3.94 µm.

### Test Example

Butyl methoxydibenzoylmethane (BMDBM) and ethylhexyl methoxycinnamate (EHMC) undergo photochemical interaction upon irradiation leading to 1:1 addition products. This results in a loss of the absorption properties, which can be measured by UV spectroscopy. Thus, this system is useful for showing the diffusion (or the retention) of EHMC from the capsules in a cosmetic emulsion. Thus, this method is suitable to prove an efficient retention of the sunscreen agent in a microcapsule.

In the following examples the photostability of the emulsions was determined initially and after storage at 43°C for several weeks according to G. Berset & H. Gonzenbach (COLIPA Task force); Proposed protocol for determination of photostability. Part I: cosmetic UV-filters, Int.J.Cosmet.Sci. 18, 167-188 (1996) using an Atlas suntes XLS+. The irradiation time was 5.5 h.

Accordingly, three reference compositions (reference A, reference B and reference C) and eight sample compositions (samples D to L) were prepared.

Reference A contains 5% EHMC, 2% BMDBM, 1.8% OC (octocrylene)

Reference B contains 5% EHMC

Reference C contains 2% BMDBM, 1.8% OC

The samples contain the corresponding amount of encapsulated EHMC, 2% BMDBM, 1.8% OC.

Sample D contains Eusolex^{®} UV-pearls™ OMC with 35% of EHMC

Sample E-K contain encapsulated EHMC, encapsulated according to WO2003066209 with varying capsule size.

The references and samples were then incorporated into a standard topical composition, and the components of the tested compositions are summarized in the following table

| | **Formula** | **Reference A %** | **Reference B %** | **Reference C %** | **Sample %** |
|---|---|---|---|---|---|
| **A)** | Glyceryl Myristate | 3.00 | 3.00 | 3.00 | 3.00 |
| | **BMDBM** | 2.00 | -- | 2.00 | 2.00 |
| | **EHMC** | 5.00 | 5.00 | -- | -- |
| | **OC** | 1.80 | -- | 1.80 | 1.80 |
| | Cetyl Alcohol | 1.00 | 1.00 | 1.00 | 1.00 |
| | Silicone 200/350 cs | 2.00 | 2.00 | 2.00 | 2.00 |
| | Tegosoft TN (=Finsolv) | 14.00 | 14.00 | 14.00 | 14.00 |
| | Amphisol A | 2.00 | 2.00 | 2.00 | 2.00 |
| | BHT | 0.05 | 0.05 | 0.05 | 0.05 |
| **B)** | Edeta BD | 0.10 | 0.10 | 0.10 | 0.10 |
| | Phenonip | 0.60 | 0.60 | 0.60 | 0.60 |
| | Tris 25 % sol. | 1.30 | 1.30 | 1.30 | 1.30 |
| | Water | 60.15 | 63.15 | 60.15 | 60.15 |
| | Propylene Glycol | 5.00 | 5.00 | 5.00 | 5.00 |
| | Carbopol ETD 2001 | 0.30 | 0.30 | 0.30 | 0.30 |
| | Tris 25 % sol. | 2.50 | 2.50 | 2.50 | 2.50 |
| **C)** | **Encapsulated EHMC** | - | - | - | d 5% EHMC |
| | | **100** | **100** | **100** | **100** |

and were prepared according to the following procedure:
Heat part A) and B) to 85°C while stirring. Add the additional non-encapsulated UV-A and/or UV-B and/or broad spectrum screen in the desired concentrations, based on their solubility, to the water or the oil phase. When homogeneous, add part B) to A) under agitation. Cool to about 45°C while stirring Then add part C). Cool to ambient temperature while stirring. Homogenize again to achieve a small particle size.

The photostability of the emulsion was determined and evaluated as indicated above. The recovery of EHMC and BMBDM in a sample has to be equal to that of the references B and C in order to prove efficient retention. If the values are decreasing over time or are equal to the values of reference A, the retention of the sunscreen agent in the microcapsule is not sufficient. The compositions were stored at 43°C to accelerate the aging process. The results are summarized in the table below.

| | Particle size | EHMC content | Measurement | Recovery after irradiation* | |
|---|---|---|---|---|---|
| | D(v, 0.5) | in capsules | after | OMC | BMDBM |
| Reference A | | | initial | 42% | 21% |
| | | | 1 week at 43°C | 44% | 22% |
| | | | 2 week at 43°C | 39% | 19% |
| | | | 4 week at 43°C | 41% | 23% |
| Reference B | | | initial | 60% | - |
| | | | 1 week at 43°C | 62% | - |
| | | | 2 week at 43°C | 59% | - |
| | | | 4 week at 43°C | 61% | - |
| Reference C | | | initial | - | 66% |
| | | | 1 week at 43°C | - | 67% |
| | | | 2 week at 43°C | - | 62% |
| | | | 4 week at 43°C | - | 65% |
| Sample D | 1.34µm | 35% | initial | 58% | 66% |
| | | | 1 week at 43°C | 56% | 53% |
| | | | 2 week at 43°C | 52% | 42% |
| | | | 4 week at 43°C | 47% | 39% |
| | | | 8 week at 43°C | 42% | 26% |
| Sample E | 1.7µm | 34% | initial | 51% | 56% |
| | | | 1 week at 43°C | 38% | 19% |
| Sample F | 2.32µm | 25% | initial | 58% | 60% |
| | | | 1 week at 43°C | 41% | 23% |
| Sample G | 3.05µm | 28% | initial | 52% | 58% |
| | | | 1 week at 43°C | 48% | 51% |
| | | | 2 week at 43°C | 47% | 52% |
| | | | 4 week at 43°C | 48% | 53% |
| Sample H | 3.8µm | 33% | initial | 52% | 58% |
| | | | 1 week at 43°C | 52% | 49% |
| | | | 2 week at 43°C | 52% | 48% |
| | | | 4 week at 43°C | 51% | 50% |
| | | | 8 week at 43°C | 53% | 51% |
| Sample J | 3.8µm | 31% | initial | 59% | 67% |
| | | | 1 week at 43°C | 62% | 66% |
| | | | 2 week at 43°C | 61%. | 68% |
| | | | 4 week at 43°C | 65% | 70% |
| | | | 8 week at 43°C | 59% | 63% |
| Sample K | 4.5µm | 36% | initial | 59% | 59% |
| | | | 1 week at 43°C | 62% | 63% |
| | | | 2 week at 43°C | 48% | 62% |
| | | | 4 week at 43°C | 59% | 58% |
| | | | 8 week at 43°C | 60% | 59% |
| Sample L | 4.2µm | 35% | initial | 58% | 65% |
| | | | 1 week at 43°C | 62% | 57% |
| | | | 2 week at 43°C | 57% | 60% |
| | | | 4 week at 43°C | 56% | 59% |
| | | | 8 week at 43°C | 59% | 60% |

| | | | | | |
|---|---|---|---|---|---|
| *measured by UV absorption of irradiated sample vs. non-irradiated sample. | | | | | |

A deviation of +/- 10% is due to the employed method and is still acceptable.

As is evident from the results above, there is a significant decrease of the recovery of EHMC and BMDBM using capsules with a particle size < 3 µm, finally leading to complete diffusion of EHMC out of the capsules. On the other side, there occurs no significant bleeding of EHMC out of the capsules having a D(v, 0.5) value of 3 µm or more, even after storage at 43° over several weeks indicating the tightness of the system.

The following table summarizes the most important conclusions which can be drawn from the results and clearly shows the unexpected retention which can be achieved, if an encapsulated sunscreen agent is used according to the invention.

| Sample | Particle size (µm) D(v, 0.5) | ∑ of change in recovery after irradiation (storage at 43°C until complete leakage or of a maximum of 8 weeks) | |
|---|---|---|---|
| | | EMHC | BMDBM |
| D* | 1.34 | -16% | -40% |
| E | 1.70 | -13% | -37% |
| F | 2.32 | -17% | -37% |
| G | 3.05 | -4% | -5% |
| H | 3.80 | +1% | -9% |
| J | 3.80 | 0% | -4% |
| K | 4.50 | +1% | +0% |
| L | 4.20 | +1% | -5% |

| | | | |
|---|---|---|---|
| *commercial Eusolex^{®} UV-pearls™ OMC | | | |

A deviation of +/-10% is due to the employed method and is still acceptable.

The encapsulated sunscreen agents of the present invention can be incorporated in various types of cosmetic or pharmaceutical light screening compositions as illustrated in the examples below:

### Example 2

O/W sun milk with pigments

| | Ingredients | INCI Nomenclature | % w / w |
|---|---|---|---|
| A) | PARSOL SLX | Polysilicone-15 | 6.00 |
| | Neo Heliopan AP | | 3.00 |
| | Tinosorb S | Hydrogenated Cocoglycerides | 3.00 |
| | Lanette O | Cetearyl Alcohol | 2.00 |
| | Myritol 318 | Caprylic/capric Triglyceride | 6.00 |
| | Mineral oil | Mineral oil | 2.00 |
| | Vitamin E acetate | Tocopheryl Acetate | 1.00 |
| | Prisorine 3515 | Isostearyl Alcohol | 4.00 |
| B) | Edeta BD | Disodium EDTA | 0.10 |
| | Phenonip | Phenoxyethanol & Methylparaben & Ethylparaben & Propylparaben & Butylparaben | 0.60 |
| | Amphisol K | Potassium Cetyl Phosphate | 2.00 |
| | Water deionized | Aqua | ad100 |
| | 1,2-Propylene Glycol | Propylene Glycol | 5.00 |
| | Carbopol 981 | Carbomer | 0.30 |
| | Tinosorb M | Methylene Bis-Benzotriazolyl Tetramethylbutylphenol | 6.00 |
| | KOH 10% solution | Potassium Hydroxide | 2.10 |
| C) | encapsulated sunscreen agent | | 1-50% |

### Procedure:

Heat part A) and B) to 85°C while stirring. When homogeneous, add part B) to A) under agitation. Cool to ambient temperature while stirring and add part C). Homogenize to achieve a small particle size.

### Example 3

Sun milk waterproofed

| | Ingredients | INCI Nomenclature | % w / w |
|---|---|---|---|
| A) | PARSOL SLX | Polysilicone-15 | 6.00 |
| | PARSOL 1789 | Butyl Methoxydibenzoylmethane | 2.00 |
| | PARSOL 5000 | 4-Methylbenzylidene Camphor | 4.00 |
| | Uvinul T 150 | Ethylhexyltriazone | 2.00 |
| | Silicone DC 200/350 cs | Dimethicone | 1.00 |
| | Lanette O | Cetearyl Alcohol | 2.00 |
| | Softisan 100 | Hydrogenated Coco-Glycerides | 3.00 |
| | Tegosoft TN | C12-15 Alkyl Benzoate | 6.00 |
| | Cetiol B | Dibutyl Adipate | 7.00 |
| | Vitamin E acetate | Tocopheryl Acetate | 2.00 |
| | BHT | BHT | 0.05 |
| | Edeta BD | Disodium EDTA | 0.10 |
| | Phenonip | Phenoxyethanol & Methylparaben & Ethylparaben & Propylparaben & Butylparaben | 0.60 |
| | Amphisol | Cetyl Phosphate DEA | 2.00 |
| B) | Water deionized | Aqua | ad 100 |
| | Propylene Glycol | Propylene Glycol | 5.00 |
| | Carbopol 980 | Carbomer | 0.30 |
| | KOH (10% sol.) | Potassium Hydroxide | 1.50 |
| C) | encapsulated sunscreen agent | | 1-50% |

### Procedure:

Heat part A) and B) to 85°C while stirring. When homogeneous, add part B) to A) under agitation. Cool to ambient temperature while stirring and add part C). Homogenize to achieve a small particle size.

### Example 4

Sun milk for babies and children

| | Ingredients | INCI Nomenclature | % w / w |
|---|---|---|---|
| A) | Titanium Dioxide | Titanium Dioxide microfine | 4.00 |
| | Tegosoft TN | C12-15 Alkyl Benzoate | 5.00 |
| | Silicone 2503 Cosmetic Wax | Stearyl Dimethicone | 2.00 |
| | Cetyl Alcohol | Cetyl Alcohol | 1.00 |
| | Butylated Hydroxytoluene | BHT | 0.05 |
| | Estol GMM 3650 | Glyceryl Myristate | 4.00 |
| | Edeta BD | Disodium EDTA | 0.10 |
| | Phenonip | Phenoxyethanol & Methylparaben & Ethylparaben & Propylparaben & Butylparaben | 0.60 |
| | Amphisol A | Cetyl Phosphate | 2.00 |
| B) | Water deionized | Aqua | ad 100 |
| | Carbopol 980 | Carbomer | 0.6 |
| | Glycerin | Glycerin | 3.00 |
| | KOH sol. 10% | Potassium Hydroxide | 2.4 |
| C) | encapsulated sunscreen agent | | 1-50% |

### Procedure:

Heat part A) and B) to 85°C while stirring. When homogeneous, add part B) to A) under agitation. Cool to ambient temperature while stirring and add part C). Homogenize to achieve a small particle size.

### Example 5

High protective sun milk

| | Ingredients | INCI Nomenclature | % w / w |
|---|---|---|---|
| A) | PARSOL SLX | Polysilicone-15 | 6.00 |
| | PARSOL 1789 | Butyl Methoxydibenzoylmethane | 2.00 |
| | PARSOL 5000 | 4-Methylbenzylidene Camphor | 4.00 |
| | Uvinul T 150 | | 2.00 |
| | Silicone DC 200/350 cs | Dimethicone | 1.00 |
| | Lanette O | Cetearyl Alcohol | 2.00 |
| | Softisan 100 | Hydrogenated Coco-Glycerides | 3.00 |
| | Tegosoft TN | C12-15 Alkyl Benzoate | 6.00 |
| | Cetiol B | Dibutyl Adipate | 7.00 |
| | Vitamin E acetate | Tocopheryl Acetate | 2.00 |
| | BHT | BHT | 0.05 |
| | Edeta BD | Disodium EDTA | 0.10 |
| | Phenonip | Phenoxyethanol & Methylparaben & Ethyl-paraben & Propylparaben & Butylparaben | 0.60 |
| | Amphisol K | Potassium Cetyl Phosphate | 2.00 |
| B) | Water deionized | Aqua | ad 100 |
| | Propylene Glycol | Propylene Glycol | 5.00 |
| | Carbopol 980 | Carbomer | 0.30 |
| | KOH (10% sol.) | Potassium Hydroxide | 1.50 |
| C) | encapsulated sunscreen agent | | 1-50% |
| D) | Perfume | Perfume | q. s. |

### Procedure:

Heat part A) and B) to 85°C while stirring. When homogeneous, add part B) to A) under agitation. Cool to ambient temperature while stirring and add part C) and D). Homogenize to achieve a small particle size.

### Example 6

Water-free sun gel

| | Ingredients | INCI Nomenclature | % w / w |
|---|---|---|---|
| A) | PARSOL MCX | Ethylhexyl Methoxycinnamate | 6.00 |
| | PARSOL 1789 | Butyl Methoxydibenzoylmethane | 4.00 |
| | PARSOL 5000 | 4-Methylbenzylidene Camphor | 4.00 |
| | Uvasorb HEB | Diethylhexyl Butamido Triazone | 1.50 |
| | Uvinul A plus | | 2.00 |
| | Vitamin E acetate | Tocopheryl Acetate | 1.50 |
| | Tegosoft TN | C12-15 Alkyl Benzoate | 9.00 |
| | Elefac I-205 | Ethylhexyldodecyl Neopentanoate | 2.00 |
| | Alcohol | Alcohol | ad 100 |
| | Isopropyl Alcohol | Isopropyl Alcohol | 20.00 |
| B) | Klucel MF | Hydroxypropylcellulose | 2.00 |
| C) | encapsulated sunscreen agent | | 1-48% |
| D) | perfume | | q.s. |

### Procedure:

Mix part A) and B) while stirring. When homogeneous, add part C) and D) under agitation.

### Example 7

Sun gel

| | Ingredients | INCI Nomenclature | % w / w |
|---|---|---|---|
| A) | Pemulen TR-2 | Acrylates/C10-30 Alky Acrylate Crosspolymer | 0.60 |
| | Phenonip | Phenoxyethanol & Methylparaben & Ethylparaben & Propylparaben & Butylparaben | 0.60 |
| | Edeta BD | Disodium EDTA | 0.1 |
| | Aqua | Aqua | ad 100 |
| B) | PARSOL 1789 | Butyl Methoxydibenzoylmethane | 4.00 |
| | PARSOL 340 | Octocrylene | 3.00 |
| | Tegosoft TN | C12-15 Alkyl Benzoate | 15.00 |
| | Antaron V-216 | PVP/Hexadecene Copolymer | 1.00 |
| | Vitamin E acetate | Tocopheryl Acetate | 0.50 |
| | Uvinul TiO2 | Titanium Dioxide | 5.00 |
| | Butylated Hydroxytoluene | BHT | 0.05 |
| | Cremophor RH 410 | PEG-40 Hydrogenated Castor Oil | 0.50 |
| | Tris Amino | Tromethamine | 0.50 |
| C) | encapsulated sunscreen agent | | 1-50% |
| D) | Perfume | Perfume | q.s. |

### Procedure:

Heat part A) and B) to 85°C while stirring. When homogeneous, add part B) to A) under agitation. Cool to ambient temperature while stirring and add part C) and D). Homogenize to achieve a small particle size.

### Example 8

High protection WO sun milk

| | Ingredients | INCI Nomenclature | % w / w |
|---|---|---|---|
| A) | PARSOL 1789 | Butyl Methoxydibenzoylmethane | 2.00 |
| | PARSOL 5000 | 4-Methylbenzylidene Camphor | 4.00 |
| | Uvinul T 150 | Ethylhexyl Triazone | 2.00 |
| | Uvinul TiO2 | Titanium Dioxide and Trimethoxycaprylylsilane | 5.00 |
| | Arlacel P 135 | PEG-30 Dipolyhydroxystearate | 2.00 |
| | Tegosoft TN | C12-15 Alkyl Benzoate | 5.00 |
| | Cosmacol EMI | Di-C12-13 Alkyl Malate | 6.00 |
| | Miglyol 840 | Propylene Glycol Dicaprylate/Dicaprate | 6.00 |
| | Butylated Hydroxytoluene | BHT | 0.05 |
| | Phenonip | Phenoxyethanol & Methylparaben & Ethylparaben & Propylparaben & Butylparaben | 0.60 |
| B) | Deionized water | Aqua | ad 100 |
| | Glycerin | Glycerin | 5.00 |
| | Edeta | Disodium EDTA | 0.1 |
| | NaCl | Sodium Chloride | 0.30 |
| C) | PARSOL HS | Phenylbenzimidazole Sulfonic Acid | 4.00 |
| | Water | Aqua | 20.00 |
| | Triethanolamine 99%. | Triethanolamine | 2.50 |
| D) | encapsulated sunscreen agent | | 1-35.45% |
| E) | Perfume | | q.s. |

### Procedure:

Heat part A), B) and C) to 85°C while stirring. When homogeneous, add part B) and C) to A) under agitation. Cool to ambient temperature while stirring and add part D) and E). Homogenize to achieve a small particle size.

### Example 9

W/O milk with pigments

| | Ingredients | INCI Nomenclature | % w / w |
|---|---|---|---|
| A) | Cremophor WO 7 | PEG-7 Hydrogenated Castor Oil | 6.00 |
| | Elfacos ST 9 | PEG-45/Dodecyl Glycol Copolymer | 2.00 |
| | PARSOL 1789 | Butyl Methoxydibenzoylmethane | 3.00 |
| | Tinosorb S | | 5.00 |
| | PARSOL 5000 | 4-Methylbenzylidene Camphor | 4.00 |
| | Uvinul TiO2 | Titanium Dioxide | 2.00 |
| | microfine ZnO | Zinc Oxide | 2.00 |
| | Microcrystalline wax | Microcrystalline Wax | 2.00 |
| | Miglyol 812 | Caprylic/capric Triglyceride | 5.00 |
| | Vitamin E acetate | Tocopheryl Acetate | 1.00 |
| | Jojoba oil | Simmondsia Chinensis Seed Oil | 5.00 |
| | Edeta BD | Disodium EDTA | 0.10 |
| | Butylated Hydroxytoluene | BHT | 0.05 |
| | Phenonip | Phenoxyethanol & Methylparaben & Ethylparaben & Propylparaben & Butylparaben | 0.60 |
| B) | Water deionized | Aqua | ad 100 |
| | Glycerin | Glycerin | 5.00 |
| C) | Neo Heliopan AP | | 2.00 |
| | Water deionized | Aqua | 20.00 |
| | KOH 10% solution | Potassium Hydroxide | 4.00 |
| D) | encapsulated sunscreen agent | | 1-31.25% |
| E) | Perfume | Perfume | q.s. |

### Procedure:

Heat part A), B) and C) to 85°C while stirring. When homogeneous, add part B) and C) to A) under agitation. Cool to ambient temperature while stirring and add part D) and E). Homogenize to achieve a small particle size.

### Example 10

Protective Day cream with Vitamin C

| | Ingredients | INCI Nomenclature | % w / w |
|---|---|---|---|
| A) | PARSOL SLX | Polysilicone-15 | 4.00 |
| | PARSOL 1789 | Butyl Methoxydibenzoylmethane | 1.50 |
| | Glyceryl Myristate | Glyceryl Myristate | 2.00 |
| | Cetyl Alcohol | Cetyl Alcohol | 0.50 |
| | Myritol 318 | Caprylic/Capric Triglyceride | 5.00 |
| | Crodamol DA | Diisopropyl Adipate | 5.00 |
| | Vitamin E acetate | Tocopheryl Acetate | 2.00 |
| | Butylated Hydroxytoluene | BHT | 0.05 |
| | Phenonip | Phenoxyethanol & Methylparaben & Ethylparaben & Propylparaben & Butylparaben | 0.60 |
| | Edeta BD | Disodium EDTA | 0.10 |
| | Amphisol K | Potassium Cetyl Phosphate | 2.00 |
| B) | Water deionized | Aqua | ad 100 |
| | 1,2-Propylene Glycol | Propylene Glycol | 2.00 |
| | D-Panthenol 75 L | Panthenol | 2.00 |
| | Ethanol | Ethanol | 5.00 |
| | Allantoin | Allantoin | 0.20 |
| | Carbopol ETD 2001 | Carbomer | 0.30 |
| | KOH 10% sol. | Potassium Hydroxide | 1.50 |
| C) | Water | Aqua | 10.00 |
| | Stay-C 50 | Sodium Ascorbyl Phosphate | 0.50 |
| D) | encapsulated sunscreen agent | | 1-50% |
| E) | Perfume | Perfume | q.s. |

### Procedure:

Heat part A), B) and C) to 85°C while stirring. When homogeneous, add part B) and C) to A) under agitation. Cool to ambient temperature while stirring and add part D) and E). Homogenize to achieve a small particle size.

## Claims

1. Topical composition containing at least a first sunscreen agent which is encapsulated and a second sunscreen agent which is different from the first sunscreen agent, **characterized in that** the first sunscreen agent is encapsulated in microcapsules having an average particle size D(v, 0.5) which meets the following condition:
3 µm ≤ average particle size D(v, 0.5) of the microcapsules ≤ 8 µm,
wherein the microcapsules are obtainable by an emulsion polymerization process or by a sol-gel process and wherein the encapsulating agent is a tetraalkoxysilane, and wherein the encapsulated sunscreen agent is either in the form of a discrete liquid or a discrete solid.

2. The topical composition according to claim 1, wherein the microcapsules meet the following condition:
3 µm ≤ average particle size D(v, 0.5) of the microcapsules ≤ 6 µm.

3. The topical composition according to claim 2, wherein the microcapsules meet the following condition:
3 µm ≤ average particle size D(v, 0.5) of the microcapsules 5 4 µm.

4. The topical composition according to any of claims 1 to 3, wherein either the first sunscreen agent or the second sunscreen agent is a cinnamic ester derivative of the general formula I 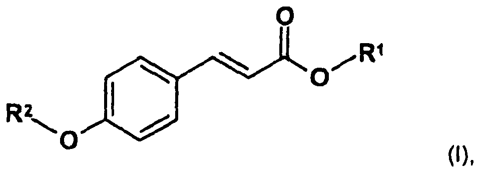 wherein R¹ and R² are independently hydrogen or saturated straight- or branched-chain alkyl containing 1 to 21 carbon atoms.

5. The topical composition according to claim 4, wherein either the first sunscreen agent or the second sunscreen agent is selected from 2-ethylhexyl methoxycinnamate, ethoxyethyl methoxycinnamate and isoamyl methoxycinnamate.

6. The topical composition according to any of claims 1 to 5, wherein the microcapsules comprise 70 to 95 wt.-% of the sunscreen agent, based on the total weight of the microcapsules.

7. The topical composition according to any of claims 1 to 6, **characterized in that** the microcapsules are obtainable by an emulsion polymerisation process, preferably an ex-situ emulsion polymerization process.

8. The topical composition according to any of claims 1 to 6, **characterized in that** the microcapsules are obtainable by an in-situ polymerisation process.

9. The topical composition according to any of claims 1 to 6, **characterized in that** the microcapsules are obtainable by a sol-gel process.

10. The topical composition according to any of claims 1 to 9, **characterized in that** either the first sunscreen agent or the second sunscreen agent is butyl methoxydibenzoylmethane.

11. The topical composition according to claim 10, **characterized in that** the second sunscreen agent is not-encapsulated and is butyl methoxydibenzoylmethane.

12. The topical composition according to any of claims 1 to 11 comprising at least one further sunscreen agent.

13. The topical composition according to claim 12 wherein the least one further sunscreen agent is selected from octocrylene and/ or polysilicones 15.

14. Encapsulated cinnamic ester derivatives of formula I 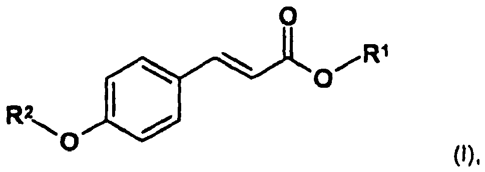 wherein R¹ and R² are independently hydrogen or saturated straight- or branched-chain alkyl containing 1 to 21 carbon atoms in the form of microcapsules, **characterized in that** the microcapsules have an average particle size D(v, 0.5) which meats the following condition:
3 µm ≤ average particle size D(v, 0.5) of the microcapsules ≤ 5 µm,
wherein the microcapsules are obtainable by an emulsion polymerization process or by a sol-gel process and wherein the encapsulating agent is a tetraalkoxysilane.

15. The encapsulated cinnamic ester derivative according to claim 14, wherein the microcapsules meet the following condition:
3 µm ≤ average particle size D(v, 0.5) of the microcapsules ≤ 4 µm.

16. The encapsulated cinnamic ester derivative according to any of claims 14 to 15, wherein the cinnamic ester derivative is selected from 2-ethylhexyl methoxycinnamate, ethoxyethyl methoxycinnamate and isoamyl methoxycinnamate.

17. The encapsulated cinnamic ester derivative according to any of claims 14 to 16 obtainable by a sol-gel process.

18. A topical composition as defined in any of claims 1 to 13 for use in a method for protecting the skin and/ or hair against UV radiation, in particular solar radiation.

19. An encapsulated sunscreen agent as defined in any of claims 14 to 17 for use in a method for protecting the skin and/ or hair against UV radiation, in particular solar radiation.

## Patentansprüche

1. Topische Zusammensetzung, enthaltend mindestens ein erstes Sonnenschutzmittel, das eingekapselt ist, und ein zweites Sonnenschutzmittel, das sich von dem ersten Sonnenschutzmittel unterscheidet, **dadurch gekennzeichnet, dass** das erste Sonnenschutzmittel in Mikrokapseln mit einer durchschnittlichen Teilchengröße D(v, 0,5), die die folgende Bedingung erfüllen:
3 µm ≤ durchschnittliche Teilchengröße D(v, 0,5) der Mikrokapseln ≤ 8 µm, eingekapselt ist, wobei die Mikrokapseln durch ein Emulsionspolymerisationsverfahren oder durch ein Sol-Gel-Verfahren erhältlich sind, und wobei das Einkapselungsmittel ein Tetraalkoxysilan ist, und wobei das eingekapselte Sonnenschutzmittel entweder in Form einer separaten Flüssigkeit oder eines separaten Feststoffes vorliegt.

2. Topische Zusammensetzung nach Anspruch 1, wobei die Mikrokapseln die folgende Bedingung erfüllen:
3 µm ≤ durchschnittliche Teilchengröße D(v, 0,5) der Mikrokapseln ≤ 6 µm.

3. Topische Zusammensetzung nach Anspruch 2, wobei die Mikrokapseln die folgende Bedingung erfüllen:
3 µm ≤ durchschnittliche Teilchengröße D(v, 0,5) der Mikrokapseln ≤ 4 µm.

4. Topische Zusammensetzung nach einem der Ansprüche 1 bis 3, wobei entweder das erste Sonnenschutzmittel oder das zweite Sonnenschutzmittel ein Zimtsäureesterderivat der allgemeinen Formel I 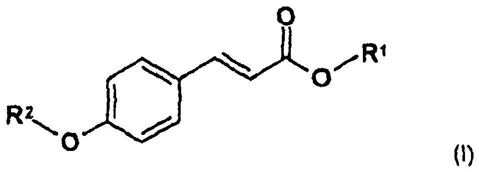 ist, wobei R¹ und R² unabhängig Wasserstoff oder gesättigtes gerad- oder verzweigtkettiges Alkyl, enthaltend 1 bis 21 Kohlenstoffatome, sind.

5. Topische Zusammensetzung nach Anspruch 4, wobei entweder das erste Sonnenschutzmittel oder das zweite Sonnenschutzmittel ausgewählt ist aus 2-Ethylhexylmethoxycinnamat, Ethoxyethylmethoxycinnamat und Isoamylmethoxycinnamat.

6. Topische Zusammensetzung nach einem der Ansprüche 1 bis 5, wobei die Mikrokapseln 70 bis 95 Gew.-% des Sonnenschutzmittels, basierend auf dem Gesamtgewicht der Mikrokapseln, umfassen.

7. Topische Zusammensetzung nach einem der Ansprüche 1 bis 6, **dadurch gekennzeichnet, dass** die Mikrokapseln durch ein Emulsionspolymerisationsverfahren, vorzugsweise ein Ex-situ-Emulsionspolymerisationsverfahren, erhältlich sind.

8. Topische Zusammensetzung nach einem der Ansprüche 1 bis 6, **dadurch gekennzeichnet, dass** die Mikrokapseln durch ein In-situ-Polymerisationsverfahren erhältlich sind.

9. Topische Zusammensetzung nach einem der Ansprüche 1 bis 6, **dadurch gekennzeichnet, dass** die Mikrokapseln durch ein Sol-Gel-Verfahren erhältlich sind.

10. Topische Zusammensetzung nach einem der Ansprüche 1 bis 9, **dadurch gekennzeichnet, dass** entweder das erste Sonnenschutzmittel oder das zweite Sonnenschutzmittel Butylmethoxydibenzoylmethan ist.

11. Topische Zusammensetzung nach Anspruch 10, **dadurch gekennzeichnet, dass** das zweite Sonnenschutzmittel nicht eingekapselt ist und Butylmethoxydibenzoylmethan ist.

12. Topische Zusammensetzung nach einem der Ansprüche 1 bis 11, umfassend mindestens ein weiteres Sonnenschutzmittel.

13. Topische Zusammensetzung nach Anspruch 12, wobei das mindestens eine weitere Sonnenschutzmittel ausgewählt ist aus Octocrylen und/ oder Polysilikon 15.

14. Eingekapselte Zimtsäureesterderivate der Formel I 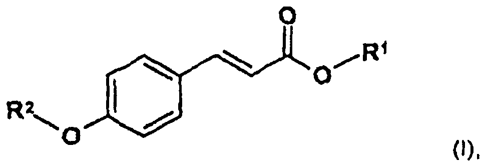 wobei R¹ und R² unabhängig Wasserstoff oder gesättigtes gerad- oder verzweigtkettiges Alkyl, enthaltend 1 bis 21 Kohlenstoffatome, sind, in Form von Mikrokapseln, **dadurch gekennzeichnet, dass** die Mikrokapseln eine durchschnittliche Teilchengröße D(v, 0,5) aufweisen, die die folgende Bedingung erfüllen:
3 µm ≤ durchschnittliche Teilchengröße D(v, 0,5) der Mikrokapseln ≤ 5 µm,
wobei die Mikrokapseln durch ein Emulsionspolymerisationsverfahren oder durch ein Sol-Gel-Verfahren erhältlich sind, und wobei das Einkapselungsmittel ein Tetraalkoxysilan ist.

15. Eingekapseltes Zimtsäureesterderivat nach Anspruch 14, wobei die Mikrokapseln die folgende Bedingung erfüllen:
3 µm ≤ durchschnittliche Teilchengröße D(v, 0,5) der Mikrokapseln ≤ 4 µm.

16. Eingekapseltes Zimtsäureesterderivat nach einem der Ansprüche 14 bis 15, wobei das Zimtsäureesterderivat ausgewählt ist aus 2-Ethylhexylmethoxycinnamat, Ethoxyethylmethoxycinnamat und Isoamylmethoxycinnamat.

17. Eingekapseltes Zimtsäureesterderivat nach einem der Ansprüche 14 bis 16, erhältlich durch ein Sol-Gel-Verfahren.

18. Topische Zusammensetzung, wie in einem der Ansprüche 1 bis 13 definiert, zur Verwendung in einem Verfahren zum Schutz von Haut und/oder Haar vor UV-Strahlung, insbesondere Sonnenstrahlung.

19. Eingekapseltes Sonnenschutzmittel, wie in einem der Ansprüche 14 bis 17 definiert, zur Verwendung in einem Verfahren zum Schutz von Haut und/oder Haar vor UV-Strahlung, insbesondere Sonnenstrahlung.

## Revendications

1. Composition topique contenant au moins un premier agent de protection solaire qui est encapsulé et un second agent de protection solaire qui est différent du premier agent de protection solaire, **caractérisée en ce que** le premier agent de protection solaire est encapsulé dans des microcapsules présentant une taille particulaire moyenne D(v, 0,5) qui satisfait à la condition suivants :
3 µm ≤ taille particulaire moyenne D(v, 0,5) des microcapsules ≤ 8 µm,
dans laquelle les microcapsules peuvent être obtenues par un procédé de polymérisation en émulsion ou par un procédé sol-gel et dans laquelle l'agent d'encapsulation est un tétraalcoxysilane, et
dans laquelle l'agent de protection solaire encapsulé se présente sous la forme d'un liquide ou d'un solide discret.

2. Composition topique selon la revendication 1, dans laquelle les microcapsules satisfont à la condition suivants :
3 µm ≤ taille particulaire moyenne D(v, 0,5) des microcapsules ≤ 6 µm,

3. Composition topique selon la revendication 2, dans laquelle les microcapsules satisfont à la condition suivante :
3 µm ≤ taille particulaire moyenne D(v, 0,5) des microcapsules ≤ 4 µm,

4. Composition topique selon l'une quelconque des revendications 1 à 3, dans laquelle le premier agent de protection solaire ou le second agent de protection solaire est un dérivé d'ester cinnamique de formule générale I suivante : 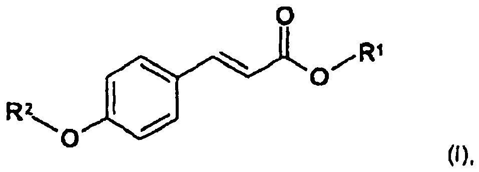 dans laquelle R¹ et R² représentent, indépendamment l'un de l'autre, l'hydrogène ou un alkyle à chaîne droite ou ramifiée, saturé, contenant 1 à 21 atomes de carbone.

5. Composition topique selon la revendication 4, dans laquelle le premier agent de protection solaire ou le second agent de protection solaire est choisi parmi le méthoxycinnamate de 2-éthylhexyle, le méthoxycinnamate d'éthoxyéthyle et le méthoxycinnamate d'isoamyle.

6. Composition topique selon l'une quelconque des revendications 1 à 5, dans laquelle les microcapsules comprennent 70 à 95 % en poids de l'agent de protection solaire, par rapport au poids total des microcapsules.

7. Composition topique selon l'une quelconque des revendications 1 à 6, **caractérisée en ce que** les microcapsules peuvent être obtenues par un procédé de polymérisation en émulsion, de préférence un procédé de polymérisation ex situ.

8. Composition topique selon l'une quelconque des revendications 1 à 6, **caractérisée en ce que** les microcapsules peuvent être obtenues par un procédé de polymérisation in situ.

9. Composition topique selon l'une quelconque des revendications 1 à 6, **caractérisée en ce que** les microcapsules peuvent être obtenues par un procédé sol-gel.

10. Composition topique selon l'une quelconque des revendications 1 à 9, **caractérisée en ce que** le premier agent de protection solaire ou le second agent de protection solaire est le méthoxydibenzoylméthane de butyle.

11. Composition topique selon la revendication 10, **caractérisée en ce que** le second agent de protection solaire n'est pas encapsulé et est le méthoxydibenzoylméthane de butyle.

12. Composition topique selon l'une quelconque des revendications 1 à 11, comprenant au moins un autre agent de protection solaire.

13. Composition topique selon la revendication 12, dans laquelle le au moins un autre agent de protection solaire est choisi parmi l'octocrylène et/ou le polysilicone 15.

14. Dérivés d'ester cinnamique encapsulés de formule I suivants : 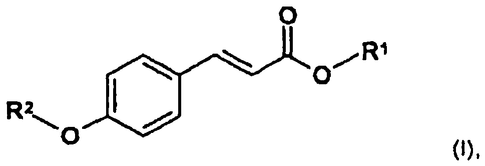 dans laquelle R¹ et R² représentent, indépendamment l'un
de l'autre, l'hydrogène ou un alkyle à chaîne droite ou ramifiée, saturé, contenant 1 à 21 atomes de carbone, sous la forme de microcapsules, **caractérisés en ce que** les microcapsules ont une taille particulaire moyenne D(v, 0,5) qui satisfait à la condition suivante :
3 µm ≤ taille particulaire moyenne D(v, 0,5) des microcapsules 5 µm,
dans lesquels les microcapsules peuvent être obtenues par un procédé de polymérisation en émulsion ou par un procédé sol-gel et dans lesquels l'agent d'encapsulation est un tétraalcoxysilane.

15. Dérivé d'ester cinnamique encapsulé selon la revendication 14, dans lequel les microcapsules satisfont à la condition-suivante .
3 µm ≤ taille particulaire moyenne D(v, 0,5) des microcapsules ≤ 4 µm,

16. Dérivé d'ester cinnamique encapsulé selon l'une quelconque des revendications 14 et 15, dans lequel le dérivé d'ester cinnamique est choisi parmi le méthoxycinnamate de 2-éthylhexyle, le méthoxycinnamate d'éthoxyéthyle et le méthoxycinnamate d'isoamyle.

17. Dérivé d'ester cinnamique encapsulé selon l'une quelconque des revendications 14 à 16, qui peut être obtenu par un procédé sol-gel.

18. Composition topique, telle que définie selon l'une quelconque des revendications 1 à 13, pour une utilisation dans un procédé de protection de la peau et/ou des cheveux contre un rayonnement UV, en particulier un rayonnement solaire.

19. Agent de protection solaire encapsulé, tel que défini selon l'une quelconque des revendications 14 à 17, pour une utilisation dans un procédé de protection de la peau et/ou des cheveux contre un rayonnement UV, en particulier un rayonnement solaire.
